# EUROPEAN PATENT APPLICATION

(11) **EP 2 535 335 A1**
(43) Date of publication of application: **19.12.2012**
(21) Application number: 11739430.4
(22) Date of filing: 07.02.2011
(51) Int. Cl.: C07D 313/20, A61K 31/352, A61P 25/28

(54) **USE OF YESSOTOXIN AND ANALOGUES AND DERIVATIVES THEREOF FOR TREATING AND/OR PREVENTING NEURODEGENERATIVE DISEASES LINKED TO TAU AND BETA AMYLOID**

(30) Priority: 08.02.2010 ES 201030162
(71) Applicant: Universidade De Santiago De Compostela, 15782 Santiago de Compostela (ES)
(72) Inventor: BOTANA LOPEZ, Luis Miguel, E-15782 Santiago de Compostela (ES); ALONSO LOPEZ, Eva, E-15782 Santiago de Compostela (ES); VALE GONZALEZ, Carmen, E-15782 Santiago de Compostela (ES)
(74) Representative: Pons Ariño, Angel
(86) International application number: PCT/ES2011/070078
(87) International publication number: WO 2011/095668

(57) **Abstract**

The present invention relates to the fields of biomedicine and pharmaceutical chemistry, specifically to the use of yessotoxin and analogues and derivatives thereof in the preparation of a medicine for preventing and/or treating neurodegenerative diseases linked to abnormal levels of tau and beta amyloid proteins, such as Alzheimer's disease.

## Description

The present invention is in the field of biomedicine and pharmaceutical chemistry. Specifically, it refers to the use of yessotoxin, its derivatives and analogues, for the preparation of a medicinal drug for the prevention and/or treatment of neurodegenerative diseases related to abnormal levels of Tau and β-amyloid proteins such as, for example, Alzheimer's disease.

### STATE OF PRIOR ART

Alzheimer's disease is a progressive neurodegenerative disease, of unknown origin, and for which no preventative or curative treatment can currently be offered. This disease affects between 5 % and 7 % of people over sixty-five years of age and is currently the most common cause of invalidity and dependence in elderly people. It is estimated that 8 million Europeans are affected by Alzheimer's disease and, taking into account the aging of the population, it is predicted that the number of sufferers will double by 2020 and triple by 2050.

This disease is characterised by a progressive loss of memory and other mental capacities as the neurones degenerate and different areas of the brain atrophy. At the neuropathological level, Alzheimer's disease is characterised by the appearance of two abnormal structures that accumulate in the brain. These structures are amyloid deposits or plaques and neurofibrillary tangles.

The amyloid deposits are insoluble fibres located intra- and extracellularly, formed by the β-amyloid (βA) peptide, more specifically by the βA40 and βA42 forms, which are generated by the sequential proteolytic rupture of the β-amyloid precursor protein (APP) by β-secretases and γ-secretases (Shirwany et al. 2007 Neuropsychiatric Disease and Treatment; 3, 597 - 612). This peptide is found in the normal form in the brain in pico or nanomolar amounts. In these amounts, the peptide is in a soluble form. When an increase in β-amyloid occurs by anomalous processing of the β-amyloid precursor protein (APP), this becomes insoluble, giving rise to the formation of deposits. Various mutations in the β-amyloid precursor protein are related to Alzheimer's disease due to an increase or abnormality in the transformation of APP into β-amyloid. In patients with Alzheimer's disease, β-amyloid aggregates appear in specific cerebral regions, inducing an inflammatory response, neuronal death and progressive cognitive deterioration. This β-amyloid peptide has also been involved in neuropathological defects in individuals with Down's syndrome.

Meanwhile, the neurofibrillary tangles by contrast are intracellular filaments formed by the polymerisation of the Tau protein, which normally acts as a protein associated with neuronal axon microtubules. These neurofibrillary tangles, which accumulate in the cytoplasm of degenerated neurones, were called "paired helical filaments" or PHFs. They show characteristics that are different from those of normal neurofilaments and microtubules. The main constituent of the PHFs is phosphorylated Tau protein. Hyperphosphorylation of Tau is due either to an increase in Tau expression, because there is a higher quantity of substrate that can be phosphorylated, or by hyperphosphorylation mediated by kinases. This abnormal protein phosphorylation of Tau is intimately related to abnormal aggregation of this protein. Tau hyperphosphorylation is currently involved in some 22 pathologies, including Alzheimer's disease, frontal lobe dementia (also called frontotemporal neurodegeneration), corticobasal degeneration, Pick's disease and Parkinson's disease with dementia.

Initially, studies were undertaken to try to elucidate the independent involvement of Tau and β-amyloid in Alzheimer's disease. The first attempts at treatment of the disease were also directed at improving the effects of each of these proteins independently. Currently, studies carried out have shown that both proteins may be related because amyloid deposits may affect different molecular pathways that facilitate Tau phosphorylation and its subsequent aggregation (Blurton-Jones et al. 2006, Current Alzheimer Research, 3 (5), 435 - 448). Furthermore, amyloid deposits may also activate various specific kinases that increase hyperphosphorylation of the Tau protein and therefore the formation of neurofibrillary tangles. Despite this relation, other studies carried out indicate that improvement in the abnormality in one of the proteins is not necessarily linked to the improvement in the other, leading in some cases to an increase in abnormality (Oddo et al., 2005. Proc Natl Acad Sci U.S.A, 102 (8), 3046 - 51). Therefore it is necessary to carry out studies in models presenting both pathologies simultaneously.

There are currently various treatments for Alzheimer's disease that do not provide a cure for the disease but act to delay its progression. The only pharmaceutical or medicinal drug approved for the treatment of the disease when it has already advanced to a moderate or severe level, that is in an advanced state, is memantine, a non-competitive antagonist of NMDA (N-methyl-D-aspartate) receptors, which prevents the toxic effect of high glutamate concentrations in neurones. Other compounds, in this case used to prevent the development of the disease, are, for example, donepezil or rivastigmine, which act by inhibiting acetylcholinesterase, increasing the levels of the neurotransmitter acetylcholine (A. Fisher 2008, Neurotherapeutics; 5: 433 - 442).
All this suggests that future studies of Alzheimer's disease and other neurodegenerative diseases will be directed towards the search for medicinal drugs acting on both abnormalities and therefore leading to complete improvement of the disease.

Currently, one of the most important sources of compounds that may be useful for the production of pharmaceuticals is the marine environment. Here a multitude of biochemical resources have been found and have been demonstrated to be very useful in healthcare such as, for example, pharmaceuticals with anti-tumour activity. Among these compounds, marine phycotoxins can have extensive clinical application because of their high diversity and, therefore, multiple cellular mechanisms of action and induced responses.

Yessotoxin was first isolated in 1986 from the scallop *Patinopecten yessoensis.* Later it was found that yessotoxin is produced by the dinoflagellates *Protoceratium reticulatum, Lingulodinium polyedrum* and *Gonyaulax spinifera.* This phycotoxin has a polyether structure, and is commonly included in the diarrheal toxins because it is extracted together with these toxins, although yessotoxins do not cause diarrhoea (Paz et al., Marine Drugs 2008, 73 - 102). Although the mechanism of action of yessotoxins has not been completely characterised, it has been reported that its main pharmacological target is activation of phosphodiesterases (Alfonso et al., Biochemical Pharmacology, 2003, 193 - 208). The compound does not exhibit oral toxicity nor has any damage to organs been described after administration of yessotoxin (Paz et al., Marine Drugs 2008, 73 - 102). Currently, there are more than 40 known analogues of yessotoxin.

Therefore, there is a need to find an effective treatment that acts on the two major elements involved in the progression of diseases such as Alzheimer's, which are the β-amyloid deposits and Tau hyperphosphorylation.

### DESCRIPTION OF THE INVENTION

Yessotoxin is a disulphated polyether compound extracted together with diarrhoeal toxins, and has the chemical structure (II). Its formula is C₅₅H₈₂O₂₁S₂Na₂. It is a compound of marine origin, produced by the dinoflagellates *Protoceratium reticulatum, Lingulodinium polyedrum* and *Gonyaulax spinifera.*

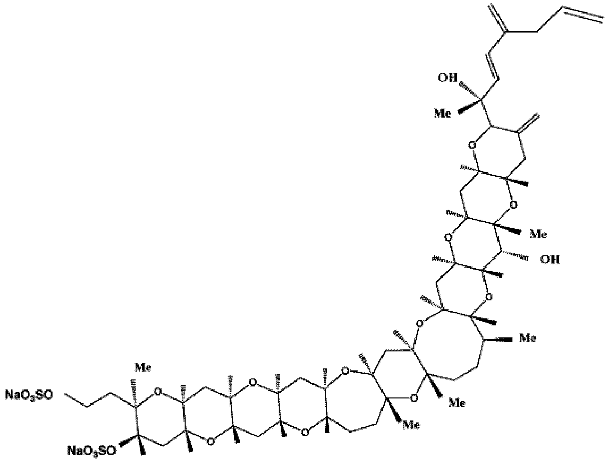

Yessotoxin has many described analogues, although the structure of some of them is still unknown. The molecular weights of yessotoxins are normally between 955 and 1551 atomic mass units. Currently, 36 natural derivatives of yessotoxin have been identified. Some of the yessotoxins are produced directly by dinoflagellates while others are produced during metabolism in the shellfish.

This molecule can be subjected to modifications giving rise to various derivatives that may have similar functionality. All these compounds, both analogues and derivatives, show a common chemical structure to that of yessotoxin (I).

Therefore, a first aspect of the present invention refers to the use of a compound of the formula (I): 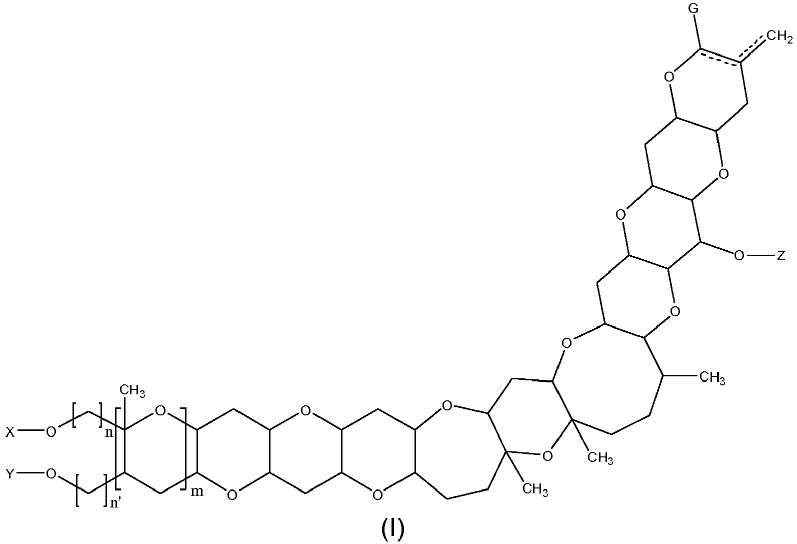 where
X and Y are independently selected from H and SO₃H,
m can be 0 or 1,
n and n' are independently selected between 0 and 5,
Z is selected from H, a monosaccharide or an oligosaccharide.
the symbol -------- represents a single or double bond,
G is a group that is selected from the groups with formula (II) to (IV): 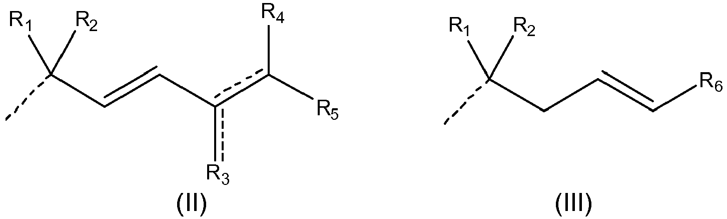 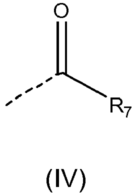 where
R₁ and R₂ are independently selected from -OH or C₁-C₅ alkyl;
R₃, R₄ and R₅ are independently selected from H, C₁-C₁₀ alkyl, C₁-C₁₀ alkenyl, OH, COOH and O;
R₆ and R₇ are independently selected from C₁-C₁₀ alkyl, C₁-C₁₀ alkenyl or amide,
or its salts, isomers or solvates,
for the manufacture of a medicinal drug for the prevention and/or treatment of neurodegenerative diseases.

The term "alkyl" in the present invention refers to radicals of hydrocarbon chains, linear or branched, that have from 1 to 10 carbon atoms, preferably from 1 to 5, and that are bound to the rest of the molecule by a single bond, for example, methyl, ethyl, n-propyl, i-propyl, n-butyl, ter-butyl, sec-butyl, n-pentyl, n-hexyl, etc. The alkyl groups can be optionally substituted by one or more substituents such as halo, hydroxy, alkoxy, carboxy, carbonyl, cyano, acyl, alkoxycarbonyl, amino, nitro, mercapto and alkylthio.

The term "alkenyl" refers to radicals of hydrocarbon chains that contain one or more double carbon-carbon bonds, for example, vinyl, 1-propenyl, allyl, isoprenyl, 2-butenyl, 1,3-butadienyl, etc. The alkenyl groups can be optionally substituted by one or more substituents such as halo, hydroxy, alkoxy, carboxy, cyano, carbonyl, acyl, alkoxycarbonyl, amino, nitro, mercapto and alkylthio.

The term "amide" refers in the present invention to a radical of the formula RCONR'R" where R, R' and R" are alkyl, alkenyl radicals or hydrogen atoms.

In a preferred embodiment, X and Y are SO₃H. In another preferred embodiment, X is H and Y is SO₃H. In another preferred embodiment, X is SO₃H and Y is H.

In a preferred embodiment, m is 1. In another preferred embodiment, m is 0. In another preferred embodiment, n is 1, 2 or 3 and n' is 0. In another preferred embodiment, n is 0 and n' is 1, 2 or 3.

In another preferred embodiment, R₁ is methyl and R₂ is OH.

In another preferred embodiment, R₃ is selected from H, C₁-C₄ alkyl, C₁-C₄ alkenyl or COOH.

In another preferred embodiment, R₄ is selected from H, OH and O.

In another preferred embodiment, R₅ is selected from H, C₁-C₄ alkyl, C₁-C₄ alkenyl or OH.

In a preferred embodiment, the present invention refers to the use of a compound with the formula (V) 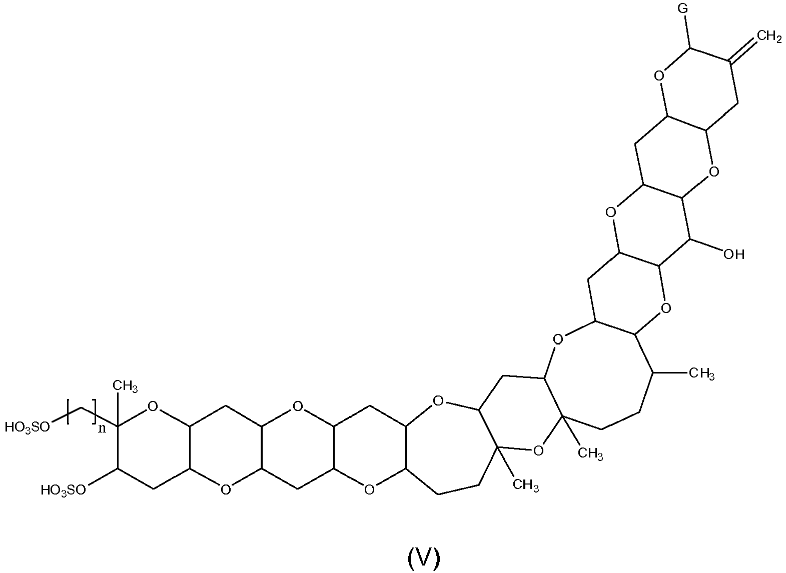 where
n is selected from 1, 2 or 3,

G is a group that is selected from the groups with formula (II) to (IV): 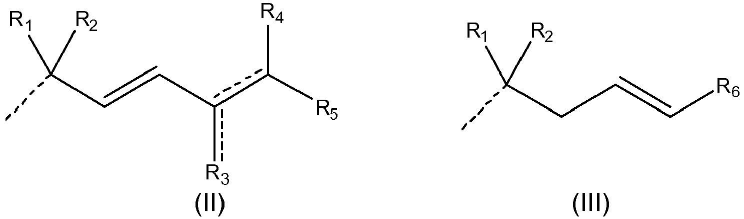 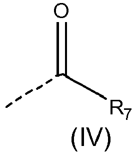 where
R₁ and R₂ are independently selected from -OH or C₁-C₅ alkyl;
R₃, R₄ and R₅ are independently selected from H, C₁-C₁₀ alkyl, C₁-C₁₀ alkenyl, - OH, -COOH and = O;
R₆ and R₇ are independently selected from C₁-C₁₀ alkyl or C₁-C₁₀alkenyl;
the symbol -------- represents a single or double bond,
or its salts, isomers or solvates,
for the manufacture of a medicinal drug for the prevention and/or treatment of neurodegenerative diseases.

In a still more preferred embodiment, G is the group with formula (II).

In still more preferred embodiment, R₁ is methyl and R₂ is OH.

In a still more preferred embodiment, R₃ is selected from H, C₁-C₄ alkyl, C₁-C4 alkenyl or COOH.

In a still more preferred embodiment, R₄ is selected from H, OH and O.

In a still more preferred embodiment, R₅ is selected from H, C₁-C₄ alkyl, C₁-C4 alkenyl and OH.

In a still more preferred embodiment, R₆ is selected from C₁-C₄ alkyl, C₁-C4 alkenyl or amide.

In a still more preferred embodiment, R₇ is a C₁-C₄ alkyl.

In another preferred embodiment, the present invention refers to the use of analogues and derivatives of yessotoxin or its salts, isomers or solvates, for the manufacture of a medicinal drug for the prevention and/or treatment of neurodegenerative diseases.

The term "analogue" as used here refers to a chemical substance similar to another chemical substance in structure and/or function. For example, the following, but not limited to, can be considered to be analogues of yessotoxin (YTX): 45-hydroxy-YTX, 45,46,47-trinor-YTX, 45,46,47-trinorhomo-YTX, homo-YTX, 45-OH-homo-YTX, carboxy-YTX, carboxyhomo-YTX, 45-OH-carboxy-YTX, noroxo-YTX (41-keto-YTX), noroxohomo-YTX (41-ketohomo-YTX), 40-epi-41-keto-YTX, 41-keto-YTX-1,3-enone, 41 a-homo-YTX, 41 a-homo-YTXamide, 44,45-diOH-YTX, 44,45-diOH-41a-homo-YTX, 45-OH-dinor-YTX, 44-oxotrinor-YTX and 41 a-homo-44-oxotrinor-YTX.

The present invention considers the term "derivative" to mean a compound that is produced starting from another by means of modifications made to the first and which has a similar functionality. Such modifications can be made, for example but without limitation, by chemical, physical, microbiological or pharmacological methods. The following, but not limited to, can be considered to be derivatives of yessotoxin:
- Desulfoderivatives: 1-desulfo-YTX, 1-desulfocarboxylhomo-YTX and 4-desulfocarboxyhomo-YTX.
- 9-methyl derivatives: 9-methyl-41-keto-YTX-1,3-enone, 9-methyl-41a-homo-YTX, 9-methyl-41a-homo-YTXamide and 44,45-diOH-9-methyl-41^{a}-homoYTX.
- Derivatives without ring A: Nor-ring-A-YTX, nor-ring-A-41-keto-YTX, nor-ring-A-40-epi-41-keto-YTX and nor-ring-A-41-keto-YTX-1,3-enone.
- 32-glycosyl derivatives: glycosylyessotoxin A (GYTX-A), protoceratin III, yessotoxin 32-O[β-L-arabinofuranosyl-( 5'→1")-β-L-arabinofuranoside, protoceratin II, Tri-glycosylyessotoxin and protoceratin IV.

The compounds of the present invention represented by the formula (I) or (V) can include isomers, depending on the presence of multiple bonds (for example, Z, E), including optical isomers or enantiomers, depending on the presence of chiral centres. The individual isomers, enantiomers or diastereoisomers and mixtures of these fall within the scope of the present invention, that is, the term isomer also refers to any mixture of isomers such as diastereoisomers, racemic mixtures, etc., including their optically active isomers or mixtures in their various proportions. Individual enantiomers or diastereoisomers, as well as their mixtures, can be separated by conventional techniques.

In a preferred embodiment, the neurodegenerative disease is related to abnormal levels of the β-amyloid proteins and/or Tau hyperphosphorylation.

Increase in β-amyloid and in phosphorylation of Tau, either separately or together, is often associated with a pathological process. These processes are fundamentally related to the nervous system because accumulation basically takes place in neurones, causing their degradation. The main pathology where these two features occur together is Alzheimer's disease. This disease progresses with an increase in β-amyloid deposits, along with hyperphosphorylation of the Tau protein giving rise to neurofibrillary tangles that cause progressive degeneration of neurones and therefore cognitive and motor deterioration. As demonstrated in the examples, yessotoxin is capable of reducing overexpression of β-amyloid and hyperphosphorylation of Tau, but does not have any effects on these structures unless they are altered. This indicates that these compounds are useful in the treatment of pathologies related to the increase in β-amyloid expression or hyperphosphorylation of Tau both independently and together.

"Pathology related to the increase of β-amyloid" in the present invention is considered to mean any pathology featuring, either an increase in the levels of the β-amyloid precursor protein or an increase in the anomalous processing of this protein, increasing the insoluble amount and therefore giving rise in both size and quantity of intra- and extra-cellular β-amyloid deposits. Included in these pathologies, for example but without limitation, are amyotrophic lateral sclerosis, Down's syndrome, vascular dementia, cerebral amyloid angiopathy related with prion proteins and Creutzfeldt-Jacob disease. Therefore, a preferred embodiment of this aspect of the invention refers to the use of a compound of chemical structure (I) for the preparation of a medicinal drug for the prevention and/or treatment of a pathology related to increase in β-amyloid that is selected from the list comprising: amyotrophic lateral sclerosis, Down's syndrome, vascular dementia, cerebral amyloid angiopathy related to prion proteins and Creutzfeld-Jacob's disease.

"Pathology related to hyperphosphorylation of Tau" in the present invention is considered to mean any pathology featuring an increase in the expression of Tau, which leads to an increase in the quantity of phosphorylated protein or a hyperphosphorylation of this protein even without change in expression, as both situations lead to an increase in the size or number of neurofibrillary tangles caused by the anomalous aggregation of phosphorylated Tau. Included in the pathologies related to Tau hyperphosphorylation are, for example but without limitation, frontotemporal dementia, progressive supranuclear paralysis, dementia associated with multiple system tauopathy, corticobasal degeneration and frontotemporal lobular degeneration or Pick's disease. Therefore, another preferred embodiment of this aspect of the invention refers to the use of a compound of chemical structure (I) for the preparation of a medicinal drug for the prevention and/or treatment of a pathology related to hyperphosphorylation of Tau that is selected from the list comprising: frontotemporal dementia, progressive supranuclear paralysis, dementia associated with multiple system tauopathy, corticobasal degeneration and frontotemporal lobular degeneration or Pick's disease.

On the other hand, there are many other diseases in addition to Alzheimer's that progress with simultaneous changes in both proteins such as, for example but not limited to, moderate cognitive disorders or deficits, hereditary cerebral hemorrhage with amyloidosis-Dutch type, cerebral amyloid angiopathy, dementia associated with Parkinson's disease, neurodegenerative disease due to diffuse Lewy bodies, corticobasal degeneration, sub-acute sclerosing panencephalitis, dementia with argyrophilic grain disease and familial Gerstmann-Straussler-Scheinker disease. Therefore, another preferred embodiment of this aspect of the invention refers to the use of a compound of chemical structure (I) for the preparation of a medicinal drug for the prevention and/or treatment of a pathology related to increase in β-amyloid and hyperphosphorylation of Tau that is selected from the list comprising: Alzheimer's disease, moderate cognitive disorders or deficits, hereditary cerebral hemorrhage with amyloidosis-Dutch type, cerebral amyloid angiopathy, dementia associated with Parkinson's disease, neurodegenerative disease due to diffuse Lewy bodies, corticobasal degeneration, sub-acute sclerosing panencephalitis, dementia with argyrophilic grain disease and familial Gerstmann-Straussler-Scheinker disease. "Moderate cognitive disorders or deficits" in the present invention is considered to mean those changes of a person's intellectual faculties that include, without limitation, deterioration of orientation, deterioration of short term memory, deterioration of reasoning, problems with calculation, problems with language, change in the ability to carry out complex tasks and change in programming ability that appear in the initial states of different diseases such as, for example but without limitation, Alzheimer's disease, schizophrenia or senile dementia.

### DESCRIPTION OF THE FIGURES

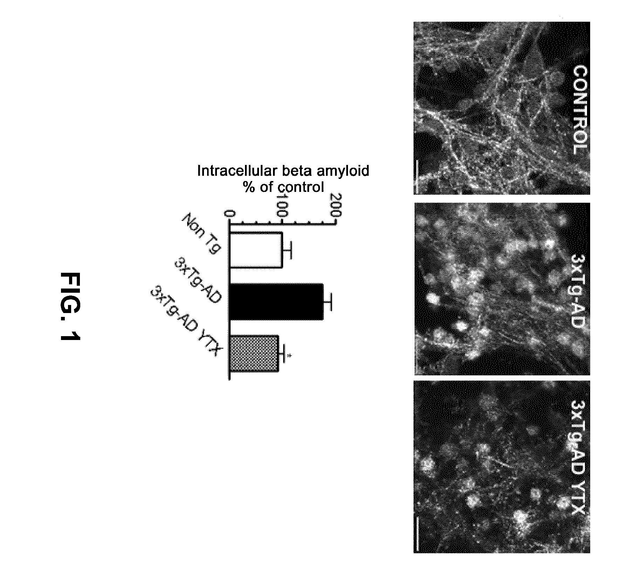

**Figure 1** shows the reduction in expression levels of intracellular β-amyloid after treatment with yessotoxin. (A) Confocal microscope images showing the expression of β-amyloid in neocortical cultures of wild animals (No Tg), neocortical cultures obtained from 3xTg-AD (3xTg) mice and levels of β-amyloid peptide in neocortical cultures of 3xTg-AD mice treated with yessotoxin (3xTg + YTX), evaluated with the 6E10 antibody. Exposure of triple-transgenic mouse cortical cultures to yessotoxin reduces overexpression of β-amyloid in this *in vitro* model. (B) Quantification of immunoreactivity showing a significant reduction of overexpression of β-amyloid after treatment with yessotoxin (**p < 0.005 compared to expression of β-amyloid in transgenic cultures, n = 3, obtained in three representative experiments, each carried out in duplicate).

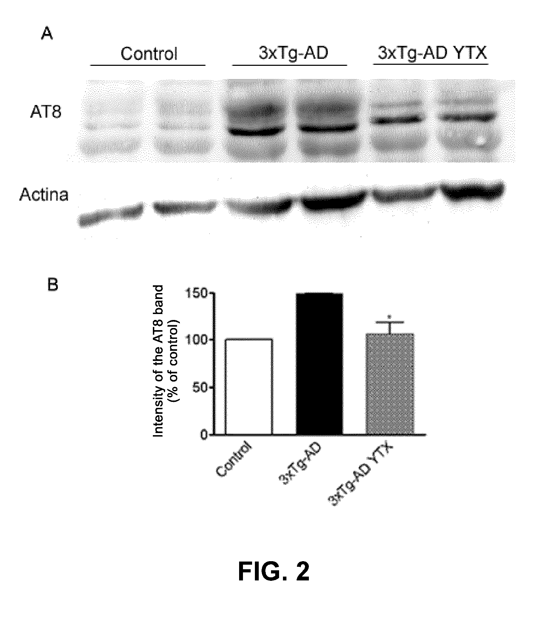

**Figure 2** shows a reduction in the levels of phosphorylated Tau in cultures of transgenic neurones treated with yessotoxin. (A) Western blot images showing levels of Tau phosphorylation using the AT8 antibody (recognises phosphorylated Tau on Ser202) in wild cultures (No Tg), transgenic cultures (3xTg) and transgenic cultures treated with yessotoxin (3xTg YTX). Data obtained in a representative experiment. (B) Quantification of the expression of phosphorylated Tau (marked with antibody AT8) showing a significant reduction of Tau phosphorylation in transgenic cultures treated with yessotoxin (*p < 0.05, n = 3 obtained in three representative experiments, each carried out in duplicate).

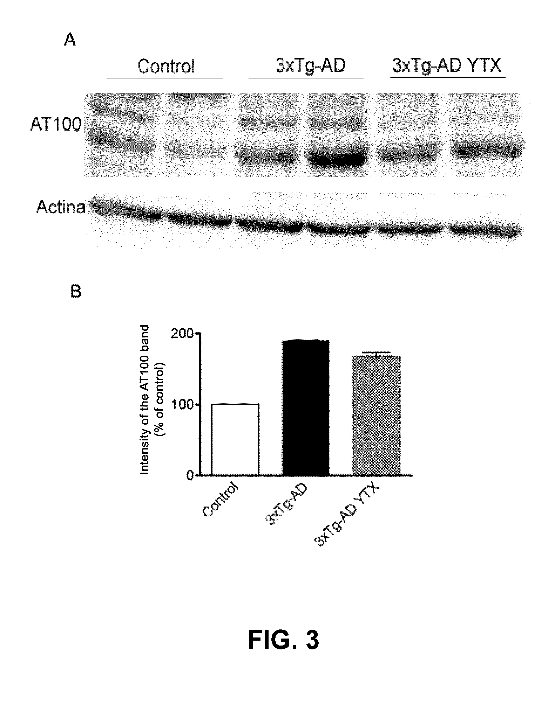

**Figure 3** shows reduction of the expression of Tau phosphorylated on residues Thr212 and Ser214 (marked with the AT100 antibody) in cultures of transgenic neurones treated with yessotoxin. (A) Western blot bands showing immunoreactivity for the AT100 antibody in wild cultures (No Tg), transgenic cultures (3xTg) and transgenic cultures treated with yessotoxin (3xTg YTX). Data obtained in a representative experiment. (B) Quantification of the expression of phosphorylated Tau (marked with antibody AT100) showing a 12 % reduction of Tau phosphorylation in transgenic cultures treated with yessotoxin (n = 3 obtained in three representative experiments, each carried out in duplicate).

### EXAMPLES

The following specific examples provided in this patent document serve to illustrate the nature of the present invention. These examples are included only for illustrative purposes and are not to be interpreted as limitations of the invention that is claimed in this document. Therefore, the examples described below illustrate the invention without limiting its field of application.

In the examples of the present invention, to see the effect of yessotoxin on the overexpression of beta-amyloid and on hyperphosphorylation of Tau, in vitro cultures of cortical neurones were used, obtained from triple-transgenic mice, which simultaneously overexpressed the human transgenes for presenilin (PS1_{M146v}), β-amyloid precursor protein (APP_{Swe}) and Tau protein (tau_{P301L}). Simultaneous overexpression of these 3 elements is related to accumulation of β-amyloid and the formation of neurofibrillary plaques and therefore these cells are useful in the study of the efficacy of compounds against diseases related to increases in Tau and β-amyloid. The examples of the present invention demonstrate that treatment with yessotoxin causes a reduction of β-amyloid and phosphorylated Tau at both the Ser202 site and the Thr212 and Ser214 sites.

### EXAMPLE 1. DETERMINATION OF THE CELLULAR VIABILITY OF TREATMENT WITH YESSOTOXIN.

In order to carry out the experiments of the present invention, either an in vitro cortical neuronal model with simultaneous overexpression of Tau and β-amyloid, obtained from a model of Alzheimer's disease in triple-transgenic (3xTg-AD or 3xTg) mice, obtainable via the detailed procedure in the international patent application WO 2003/053136 and provided by the title holders of this application, or an in vitro cortical neuronal model obtained from non-transgenic (no Tg) mice was used. The triple-transgenic neuronal model showed overexpression of presenilin (PS1_{M146v}), β-amyloid precursor protein (APP_{Swe}) and Tau protein (tau_{P301L}), which gives rise to a model of Alzheimer's disease with overexpression of β-amyloid and hyperphosphorylation of Tau.

The primary cortical cultures in the present invention were obtained from 3xTg-AD mouse embryos of 15-17 days gestation and the wild cultures were obtained from non-3xTg control mouse embryos of the same strain. The effect of the compounds used in this invention on cellular viability was estimated by a fluorescence assay using the Alamar Blue viability indicator. To carry out the assay, primary cortical cultures seeded on 96-well plates were used. The neuronal cells were incubated with yessotoxin, which was added to the culture medium at different concentrations, and the effect on in vitro neuronal viability at different treatment times was determined. The maximum concentration of yessotoxin evaluated was 1 nM and the concentration of Alamar blue was 10 %. The volume of the reaction used was 200 µl. Fluorescence was measured at wavelengths of 530 nm (excitation) and 590 nm (emission). Yessotoxin did not affect in vitro cell viability up to concentrations of 1 nM.

### EXAMPLE 2. EFFECT OF YESSOTOXIN ON OVEREXPRESSION OF INTRACELLULAR β-AMYLOID AND TAU HYPERPHOSPHORYLATION

The effect of yessotoxin on overexpression of β-amyloid and Tau hyperphosphorylation was determined by immunocytometric and Western blot techniques. Primary neuronal cultures were treated with 1 nM yessotoxin for between 3 and 7 days of culture. The cells were then processed following the usual protocols for immunocytometry and Western blot. For immunocytometric and Western blot studies, protein expression was evaluated using 6E10 anti-β-amyloid primary antibodies at a dilution of 1: 500, AT8 anti-Tau (Tau phosphorylated on Ser202, dilution 1: 1000) and AT100 anti-Tau (Tau phosphorylated on Thr212 and Ser214, dilution 1: 1000). For Western blot assays, the neuronal cultures treated with yessotoxin were washed with cold phosphate buffer and lysed in 50 mM Tris-HCl buffer (pH 7.4) containing 150 mM NaCl, 1 mM EDTA, 1 % Triton X-100, 2 mM DTT, 2.5 mM PMSF, 40 mg/ml aprotinin, 4 mg/ml leupeptin, 5 mM NaF, 1mM Na₃VO₄, 1 mg/ml pepstatin A and 1 mg/ml benzamidine. Total protein concentration was determined by Bradford's method using bovine albumin as standard. Aliquots of cellular lysates containing 20 µg total protein were loaded in loading buffer (50 mM Tris-HCl, 100 mM dithiothreitol, 2 % SDS, 20 % glycerol, 0.05 % bromophenol blue, pH 6.8); the proteins were separated by electrophoresis and transferred to PVDF membranes. The membranes were incubated with the primary antibodies, washed, and then incubated with a secondary antibody linked to HRP; the immunoreactivity was detected by chemiluminescence. The same membranes were reincubated with an anti-β-actin primary antibody to carry out data correction for the sample protein content.

For immunocytometric studies, the neuronal cultures were washed with phosphate buffer, fixed with 4 % paraformaldehyde and incubated with the primary antibody overnight. Next, the neuronal cultures were washed again with phosphate buffer and incubated with the secondary antibody for 2 hours. Then the cells were washed and mounted in mounting liquid. In immunocytometric assays, immunoreactivity was seen using a secondary fluorescent antibody in a confocal microscope (Nikon, Melville, NY, USA) with a Hamamatsu ORCA-ER camera (Hamamatsu Photonics KK, Hamamatsu, Japan).

In these assays, treatment with yessotoxin was demonstrated to reduce overexpression of β-amyloid (Fig. 1) and Tau phosphorylated on the Ser202 (Fig. 2) and on the Thr212 and Ser214 (Fig 3) sites in neurones obtained from triple transgenic mice.

## Claims

1. Use of a compound with the formula (I) 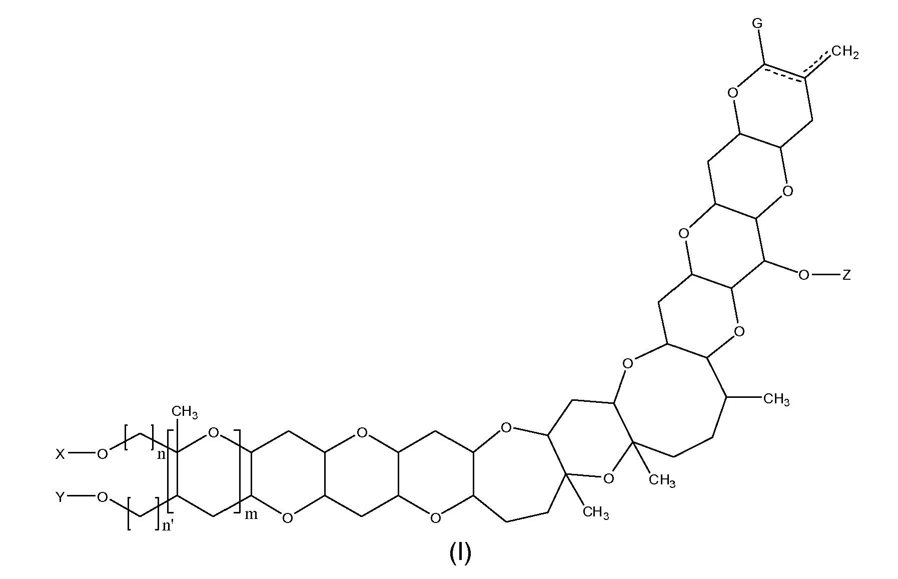 where
X and Y are independently selected from H and SO₃H,
m can be 0 or 1,
n and n' are independently selected between 0 and 5,
Z is selected from H, a monosaccharide or an oligosaccharide,
the symbol -------- represents a single or double bond,
G is a group that is selected from the groups with formula (II) to (IV): 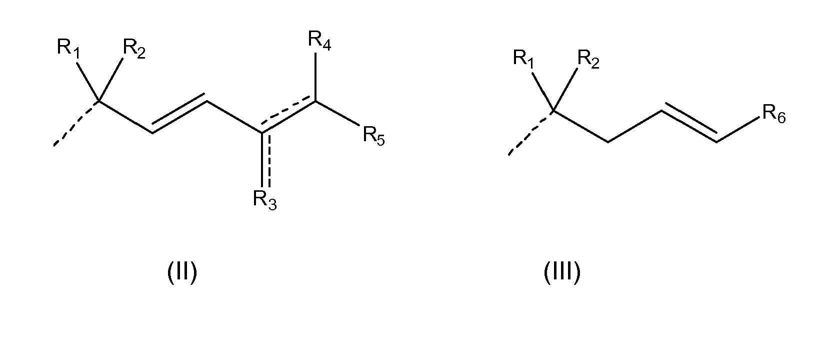 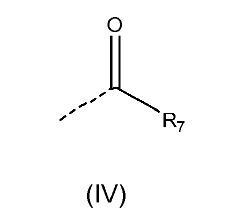 where
R₁ and R₂ are independently selected from -OH or C₁-C₅ alkyl;
R₃, R₄ and R₅ are independently selected from H, C₁-C₁₀ alkyl, C₁-C₁₀ alkenyl, - OH, COOH and O;
R₆ and R₇ are independently selected from C₁-C₁₀ alkyl, or C₁-C₁₀ alkenyl, amide,
or its salts, isomers or solvates,
for the manufacture of a medicinal drug for the prevention and/or treatment of neurodegenerative diseases.

2. Use according to claim 1 wherein X and Y are SO₃H.

3. Use according to claim 1 wherein X is H and Y is SO₃H.

4. Use according to claim 1 wherein X is SO₃H and Y is H.

5. Use according to any of the claims 1 to 5 wherein m is 1.

6. Use according to any of the claims 1 to 5 wherein m is 0.

7. Use according to any of the claims 1 to 6 wherein n is 1, 2 or 3 and n' is 0.

8. Use according to any of the claims 1 to 6 wherein n is 0 and n' is 1, 2 or 3.

9. Use of a compound according to any of the claims 1 to 8 wherein R₁ is methyl and R₂ is OH.

10. Use of a compound according to any of the claims 1 to 9 wherein R₃ is selected from H, C₁-C₄ alkyl, C₁-C₄ alkenyl, or COOH.

11. Use of a compound according to any of the claims 1 to 10 wherein R₄ is selected from H, OH or O.

12. Use of a compound according to any of the claims 1 to 11 wherein R₅ is selected from H, C₁-C₄ alkyl, C₁-C₄ alkenyl or OH.

13. Use of a compound with the formula (V) 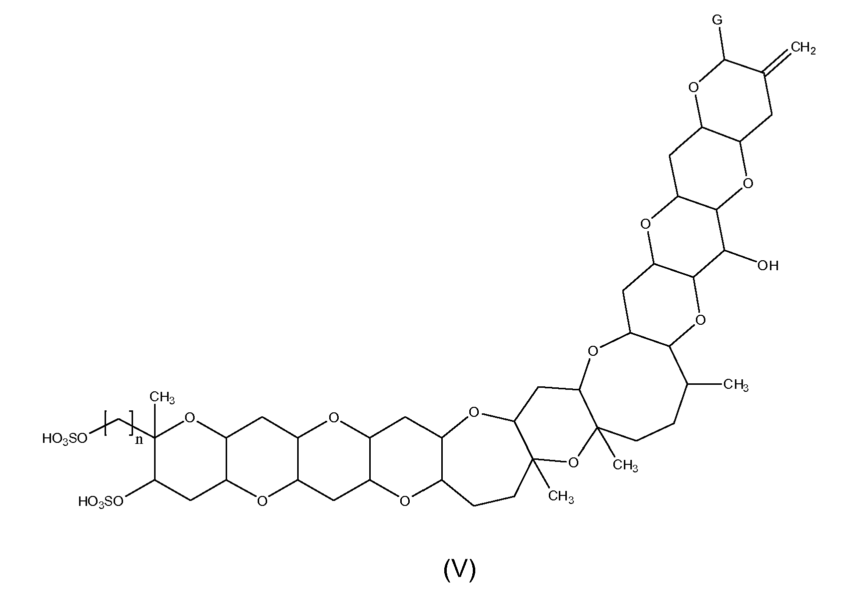 where
n is selected from 1, 2 or 3,
G is a group that is selected from the groups with formula (II) to (IV): 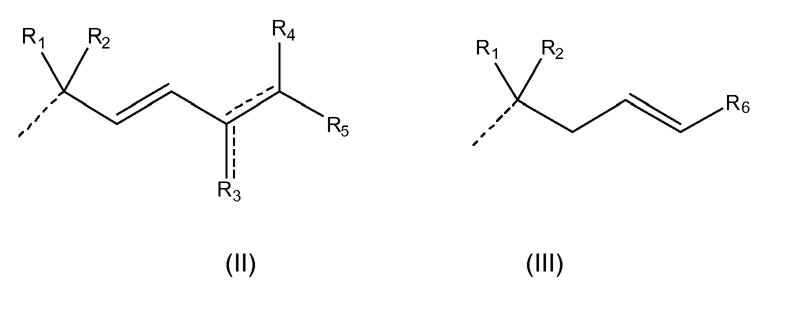 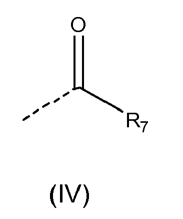 where
R₁ and R₂ are independently selected from -OH or C₁-C₅ alkyl;
R₃, R₄ and R₅ are independently selected from H, C₁-C₁₀ alkyl, C₁-C₁₀ alkenyl, - OH, -COOH and = O;
R₆ and R₇ are independently selected from C₁-C₁₀ alkyl or C₁-C₁₀ alkenyl;
the symbol -------- represents a single or double bond,
or its salts, isomers or solvates,
for the manufacture of a medicinal drug for the prevention and/or treatment of neurodegenerative diseases.

14. Use according to claim 13 wherein G is a group with the formula (II).

15. Use of a compound according to any of the claims 13 or 14 wherein R₁ is methyl and R₂ is OH.

16. Use of a compound according to any of the claims 13 to 15 wherein R₃ is selected from H, C₁-C₄ alkyl, C₁-C₄ alkenyl or COOH.

17. Use of a compound according to any of the claims 13 to 16 wherein R₄ is selected from H, OH or O.

18. Use of a compound according to any of the claims 1 to 17 wherein R₅ is selected from H, C₁-C₄ alkyl, C₁-C₄ alkenyl and OH.

19. Use of a compound according to any of the claims 1 to 18 wherein R₆ is selected from C₁-C₄ alkyl, C₁-C₄ alkenyl or amide.

20. Use of a compound according to any of the claims 1 to 19 wherein R₇ is a C₁-C₄ alkyl.

21. Use of a compound that is selected from the list comprising: Yessotoxin (YTX), 45-hydroxy-YTX, 45,46,47-trinor-YTX, 45,46,47- trinorhomo-YTX, Homo-YTX, 45-OH-homo-YTX, Carboxy-YTX, Carboxyhomo-YTX, 45-OH-carboxy-YTX, noroxo-YTX, noroxohomo-YTX, 40-epi-41-keto-YTX, 41-keto-YTX-1,3-enone, 41a-homo-YTX, 41a-homo-YTXamide, 44,55-doOH-41a-homo-YTX, 45-OH-dinor-YTX, 44-oxotrinor-YTX, 41 a-homo-44-oxotrinor-YTX, or its salts, isomers or solvates for the manufacture of a medicinal drug for the prevention and/or treatment of neurodegenerative diseases.

22. Use of a compound that is selected from the list comprising: 1-desulfo-YTX, 1-desulfocarboxyhomo-YTX, 4-desulfocarboxyhomo-YTX or its salts, isomers or solvates for the manufacture of a medicinal drug for the prevention and/or treatment of neurodegenerative diseases.

23. Use of a compound that is selected from the list comprising: 9-methyl-41-keto-YTX-1,3-enone, 9-methyl-41a-homo-YTX, 9-methyl-41a-homo-YTXamide, 44,55-diOH-9-methyl-41a-homo-YTX or their salts, isomers or solvates for the manufacture of a medicinal drug for the prevention and/or treatment of neurodegenerative diseases.

24. Use of a compound that is selected from the list comprising: Nor-ring-A-YTX, nor-ring-A-41-keto-YTX, nor-ring-A-40-epi-41-keto-YTX and nor-ring-A-41-keto-YTX-1,3-enone or their salts, isomers or solvates for the manufacture of a medicinal drug for the prevention and/or treatment of neurodegenerative diseases.

25. Use of a compound that is selected from the list comprising: glycosylyessotoxin A (GYTX-A), Protoceratin III, Yessotoxin 32-O-[β-L-arabinofuranosyl-(5'→1")-β-L-arabinofuranoside, Protoceratin II, Tri-glycosylyessotoxin and Protoceratin IV for the manufacture of a medicinal drug for the prevention and/or treatment of neurodegenerative diseases.

26. Use of a compound according to any of the claims 1 to 25 wherein the neurodegenerative disease is related to abnormal levels of the β-amyloid proteins and/or Tau hyperphosphorylation.

27. Use of a compound according to claim 26 wherein the disease related to the β-amyloid increase is selected from the list comprising: amyotrophic lateral sclerosis, Down's syndrome, vascular dementia, cerebral amyloid angiopathy related to prion proteins and Creutzfeld-Jacob's disease.

28. Use of a compound according to claim 26 wherein the disease related to Tau hyperphosphorylation is selected from the list comprising: frontotemporal dementia, progressive supranuclear paralysis, dementia associated with multiple system tauopathy, corticobasal degeneration and frontotemporal lobular degeneration or Pick's disease.

29. Use of a compound according to claim 26 wherein the disease related to the increase in β-amyloid and Tau hyperphosphorylation is selected from the list comprising: Alzheimer's disease, moderate cognitive disorders or deficits, hereditary cerebral hemorrhage with amyloidosis-Dutch type, cerebral amyloid angiopathy, dementia associated with Parkinson's disease, neurodegenerative disease due to diffuse Lewy bodies, corticobasal degeneration, sub-acute sclerosing panencephalitis, dementia with argyrophilic grain disease and familial Gerstmann-Straussler-Scheinker disease.
